# FASCICULE DE BREVET EUROPEEN

(11) **EP 3 355 866 B1**
(45) Date de publication et mention de la délivrance du brevet: **18.03.2020**
(21) Numéro de dépôt: 16770938.5
(22) Date de dépôt: 27.09.2016
(51) Int. Cl.: A61K 9/12, A61K 9/46, A61K 31/573, A61P 17/06

(54) **COMPOSITION NETTOYANTE AUTO-MOUSSANTE CONTENANT DU PROPIONATE DE CLOBÉTASOL, ET SON UTILISATION DANS LE TRAITEMENT DU PSORIASIS**
SELBSTSCHÄUMENDE REINIGUNGSZUSAMMENSETZUNG MIT CLOBETASOLPROPIONAT UND VERWENDUNG DAVON BEI DER BEHANDLUNG VON PSORIASIS
SELF-FOAMING CLEANSING COMPOSITION CONTAINING CLOBETASOL PROPIONATE, AND USE THEREOF IN THE TREATMENT OF PSORIASIS

(30) Priorité: 29.09.2015 FR 1559207
(43) Date de publication de la demande: 08.08.2018
(73) Titulaire: Galderma Research & Development, 06410 Biot (FR)
(72) Inventeur: BUGE, Jean-Christophe, 06200 Nice (FR); NADAU-FOURCADE, Karine, 06270 Villeneuve Loubet (FR)
(74) Mandataire: Casalonga
(86) Numéro de dépôt international: PCT/EP2016/073011
(87) Numéro de publication internationale: WO 2017/055294

(56) Documents cités:
- WO-A1-00/27356
- WO-A1-2014/201541
- FR-A1- 2 761 600
- US-A1- 2004 184 992
- US-A1- 2011 008 267
- US-A1- 2013 244 976

## Description

La présente invention a pour objet un produit topique nettoyant rincé sous forme d'une mousse pour le traitement pharmaceutique de la peau, efficace notamment pour le traitement du psoriasis du cuir chevelu, et contenant du propionate de clobétasol.

Le psoriasis est une des maladies de la peau les plus fréquentes parmi toutes les maladies chroniques de la peau. Le cuir chevelu est l'un des sites privilégiés pour le psoriasis; ce dernier provoque essentiellement des érythèmes, desquamations, hyperkératoses ou prurits et peut également être responsable d'une diminution de la densité des cheveux. Les traitements utilisés à ce jour contiennent de l'acide salicylique, les stéroïdes locaux, du goudron. Ces traitements sont déplaisants, notamment l'application de goudron, et nécessitent de longues applications, notamment lorsque des pommades capillaires sont utilisées.

Dans le but d'améliorer la qualité de vie du patient, sans toutefois diminuer l'effet thérapeutique du traitement, des compositions moussantes contenant des corticostéroïdes ont été développées. Plus particulièrement, le temps d'application de ces compositions moussantes est réduit par rapport au traitement classique.
Le propionate de clobétasol est un dermocorticoïde de classe 1, à savoir un corticoïde de très forte activité. Comme les autres dérivés de la cortisone à usage local, il freine le renouvellement et la multiplication des cellules de la peau. Son efficacité sur le psoriasis et particulièrement le psoriasis du cuir chevelu est avéré.

Il existe le besoin de disposer de nouvelles formes galéniques de produit nettoyant moussant dans lesquelles le propionate de clobétasol est stable, bien tolérée, efficace et agréable à appliquer.

Malgré les nouvelles générations de tensio-actifs moussants, les produits d'hygiène et tout particulièrement les produits nettoyants restent irritants et peuvent entraîner une tolérance médiocre du produit. Cependant, l'utilisateur associe le volume et la quantité de mousse au pouvoir nettoyant et à l'efficacité de celui-ci. Il existe donc un besoin de mettre au point de nouvelles formes galéniques moussantes contenant peu ou pas de tensio-actifs moussants qui soient à la fois efficaces et respectent les attentes de l'utilisateur, tout en améliorant la tolérance de ce genre de produit.

En effet, de manière générale, les compositions moussantes contiennent une quantité importante de tensio-actifs moussants. Cette forte teneur génère de l'irritation cutanée. La présente invention vise à proposer une composition qui soit particulièrement bien tolérée, comme le montrent les exemples illustrant une des méthodes d'évaluation de la tolérance présentés ci-après.
Il existe différentes méthodes d'évaluation de la tolérance d'un produit pharmaceutique ou cosmétique à usage cutané parmi lesquels on peut citer le test in vivo « in used » or « human patch test », mais aussi le test in vitro tel que le test de mesure de l'irritation sur Epiderme humain reconstruit (Reconstructed Human Epidermis ou RHE) décrit dans le protocole TG439 OCDE. Cette dernière méthode est détaillée dans l'exemple 3.

Il existe actuellement des mousses ou compositions moussantes nettoyantes sur le marché. Toutefois, elles présentent toutes un certain nombre d'inconvénients.
En effet, il existe quatre types de mousses ou compositions moussantes nettoyantes:
- Des aérosols dans lesquels la mousse est générée par un gaz propulseur mais avec l'inconvénient d'être des aérosols présentant les risques bien connus de ceux-ci (pollution, risques respiratoires notamment).
- Des formules riches en tensioactifs moussants. Ces formules présentent l'inconvénient d'être dans le meilleur des cas légèrement irritantes, le plus souvent elles sont irritantes. De plus, les actifs sensibles à la présence de tensioactifs moussants en quantité assez importante ne peuvent pas être envisagés dans ce type de composition.
- Des crèmes foisonnées rinçables dans lesquelles des bulles d'air sont introduites dans le produit grâce à un procédé de fabrication particulier. Ce procédé de fabrication présente l'inconvénient d'être très contraignant au niveau industriel et nécessite un investissement lourd au niveau du matériel industriel de conditionnement.
- Des formules moussantes peu riches en tensioactifs moussants mais conditionnées dans un packaging/emballage muni d'un système mécanique générateur de mousse (pompe avec grille de type pulvorex). Ce type de packaging présente l'inconvénient de n'être compatible qu'avec les formes galéniques très fluides.

Ainsi, il subsiste donc le besoin de mettre au point une composition pharmaceutique dont la forme galénique est différente des formes galéniques connues afin de pallier leurs inconvénients et de permettre ainsi l'utilisation du propionate de clobétasol sous forme solubilisé dans des compositions moussantes, nettoyantes, rinçables et bien tolérées destinées à une application topique chez l'être humain.

Le but de la présente invention est donc de proposer une telle composition répondant à ces besoins.

La demanderesse a ainsi mis au point une nouvelle composition pharmaceutique, destinée à une application topique rincée qui se présente sous la forme d'une composition auto-moussante (de préférence un shampooing auto-moussant) avec peu de tensioactif moussant, c'est-à-dire avec une teneur en tensioactif moussant qui soit avantageusement inférieure ou égale à 2,5% en poids de matière active par rapport au poids de la composition totale.

On entend par tensioactif moussant des tensioactifs qui produisent une mousse volumineuse, stable et onctueuse lorsqu'ils sont mélangés à l'eau selon les tests bien connus de l'homme du métier.

Constituent notamment des tensioactifs moussants : les tensioactifs anioniques, les tensioactifs cationiques, les tensioactifs amphotères et les tensioactifs non ioniques de la famille des alkylpolyglucosides et des glucamides.

La forme galénique selon l'invention présente l'avantage de garantir une bonne stabilité du propionate de clobetasol. De plus, cette formulation conduit avantageusement à l'obtention d'une mousse douce, parfaitement tolérée et non irritante, qui permet le traitement et le nettoyage du cuir chevelu tout en palliant les problèmes de tolérance et en satisfaisant le client en matière de qualité de mousse.

Enfin, de manière avantageuse, cette forme galénique ne nécessite pas pour sa mise en oeuvre l'utilisation de gaz propulseurs ou d'aérosols. Ainsi les mousses dites aérosol ou spray sont exclues du champ de l'invention. De même, les compositions moussantes rinçables de l'art antérieur de type compositions moussantes classiques riches en tensioactifs moussants et/ou les formules moussantes à teneur plus réduites en tensioactifs mais nécessitant un système mécanique générateur de mousse (type pulvorex) sont également exclues de l'invention. Il en va de même des compositions moussantes faisant intervenir un procédé de foisonnage.

La présente invention a enfin pour objet un médicament destiné à une application topique sur la peau et le cuir chevelu, comprenant une telle composition. Selon l'invention, après son application la composition est éliminée par rinçage.

La présente invention a également pour objet la composition selon l'invention, pour son utilisation dans le traitement du psoriasis.

La présente invention sera décrite plus en détail dans la description et les exemples ci-après. Dans la description qui suit, sauf indication expresse les teneurs des ingrédients sont exprimées en termes de quantité de matière active.

Cette composition est capable de prendre la forme d'une mousse par le seul fait de sa composition et peut ainsi être également définie comme une composition auto-moussante pour application topique.

La présente invention a, en conséquence, pour premier objet une composition contenant du propionate de clobétasol destinée à une application topique se présentant sous la forme d'une mousse, avantageusement de consistance semi-solide, contenant de préférence peu de tensioactif moussant (teneur inférieure ou égale à 2,5% en poids, par rapport au poids de la composition totale), et comprenant un milieu pharmaceutiquement compatible avec une application topique rincée notamment sur la peau et le cuir chevelu. Par composition se présentant sous la forme d'une mousse, (également dénommée ci-après composition auto-moussante), il est entendu une composition de consistance semi-solide ayant une forme aérée assimilable à une mousse.

Préférentiellement, l'agent générateur de gaz est présent dans l'une des formules intermédiaires citées précédemment.

La composition auto-moussante selon la présente invention comprend au moins deux compositions ou formules intermédiaires :
- au moins une composition intermédiaire B comprenant un agent générateur de gaz et au moins un agent gélifiant et/ou agent de suspensions choisi parmi les polymères d'acide acrylique, les polysaccharides, la famille des aluminium magnésium silicates la famille des amidons modifiés et la famille des carraghénanes,
- au moins une composition intermédiaire A comprenant un agent activateur de l'agent générateur de gaz et au moins un agent gélifiant et/ou agent de suspensions choisi parmi les polysaccharides, la famille des aluminium magnésium silicates, la famille des amidons modifiés, la famille des carraghénanes et le PVA, et
- du propionate de clobétasol contenu dans l'une au moins desdites formules intermédiaires A et B.

De préférence, le propionate de clobétasol est contenu dans la composition intermédiaire A.

La composition selon l'invention est auto-moussante, c'est-à-dire qu'elle mousse par simple mélange des compositions intermédiaires A et B. La présente invention a également pour objet la composition sous forme de mousse résultant du mélange desdites compositions intermédiaires A et B.

En fonction des agents présents dans la composition intermédiaire (ou formule intermédiaire), ainsi que de leurs proportions dans ladite composition, celle-ci peut se présenter sous toutes les formes galéniques ou prendre toutes les textures connues utilisables en pharmacie, pour une application topique.

De manière préférée, chaque composition intermédiaire (ou formule intermédiaire) selon l'invention peut donc se présenter par exemple sous forme de gel, émulsion (crème, crème sans tensioactif, lotion, lait ou crème fluide), sérum, solution, suspension, et préférentiellement sous la forme d'émulsion (crème, crème sans tensioactif, lotion, lait ou crème fluide) ou de gel. L'ensemble de ces formulations entrent dans le cadre de l'invention.

Selon l'invention, chaque composition (ou formule) intermédiaire peut présenter une viscosité (mesurée à 25°C et à pression atmosphérique) comprise entre 1cP et 500000 cP, avantageusement entre 500 cP et 350000 cP, mesurée avec une méthode classique de type brookfield RV DV II : Aiguille 6 vit 2.

Selon l'invention, on entend par agent activateur de l'agent générateur de gaz un ingrédient qui, par réaction chimique avec l'agent générateur de gaz, libère un gaz. Préférentiellement, il s'agit d'une réaction acide/base.

Selon l'invention le gaz généré par l'agent générateur de gaz peut être tout gaz physiologiquement compatible et permettant l'obtention d'une mousse, comme par exemple le dioxyde de carbone (CO₂) ou l'oxygène (O₂). De préférence, le gaz généré à partir de l'agent générateur de gaz est du dioxyde de carbone (CO₂).

Selon l'invention, la concentration en gaz pouvant varier, la quantité de bulles dans la composition peut varier et ainsi donner une composition pouvant aller de peu aérée à très fortement aérée.

Ainsi selon l'invention, la composition auto-moussante peut se présenter préférentiellement sous toutes les formes allant de aérée à une mousse très expansée.

La composition selon l'invention est adaptée à une application topique et peut comprendre en outre un milieu physiologiquement acceptable, c'est-à-dire un milieu compatible avec la peau et les phanères. Il s'agit de préférence d'un milieu pharmaceutiquement acceptable.

En outre, la composition peut comprendre tout agent actif susceptible de présenter une activité, éventuellement thérapeutique. Ces agents actifs peuvent être, entre autres, choisis parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

Selon l'invention, la composition sous forme de mousse (c'est-à-dire prête à être appliquée) présente un pH compris entre 2 et 8, préférentiellement entre 3 et 7.

Dans la mesure où la ou les composition(s) (ou formule(s)) intermédiaire(s) nécessite(nt) un stockage en au moins 2 compartiments pour des raisons de stabilité des ingrédients, la présente invention se rapporte soit à un unique contenant compartimenté (chaque compartiment accueillant une formule intermédiaire) et de préférence comprenant 2 ou 3 compartiments, soit à un kit comprenant chaque formule intermédiaire stockée indépendamment l'une de l'autre et physiquement séparées.
Le mélange intime extemporané (directement sur la peau ou sur tout autre support) des formules intermédiaires permet d'obtenir la composition sous forme de mousse selon l'invention.

De manière plus spécifique, la composition (ou formule) intermédiaire A peut se présenter sous forme d'une solution, d'une émulsion (lotion, crème, crème sans émulsionnant, lait, crème fluide) ou d'un gel. Cette composition contient avantageusement l'agent activateur de l'agent générateur de gaz, préférentiellement un acide, en quantité suffisante (pouvant se présenter sous forme d'un tampon acide/base à pH acide) qui peut être à titre d'exemple non limitatif le couple acide citrique/citrate de sodium.

La formule B peut se présenter sous forme d'une solution, d'un gel, d'une émulsion (lotion, crème, crème sans émulsionnant, lait, crème fluide). Cette composition contient avantageusement en quantité suffisante un agent générateur de gaz, qui peut être en particulier du bicarbonate de sodium.

Ainsi, l'invention a également pour objet un kit ou un contenant unique à plusieurs compartiments tel que défini précédemment, permettant la préparation extemporanée d'une composition sous forme de mousse selon l'invention, comprenant de manière séparée au moins deux formules intermédiaires (ou compositions intermédiaires):
- une formule intermédiaire A telle que définie ci-avant et comprenant au moins un agent activateur de l'agent générateur de gaz; et
- une formule intermédiaire B telle que définie ci-avant et comprenant au moins un agent générateur de gaz ;
- du propionate de clobétasol étant contenu dans l'une au moins desdites formules intermédiaires A et B.

De préférence, le propionate de clobétasol est contenu dans la composition intermédiaire A.

### AGENT ACTIVATEUR DE GAZ :

L'agent activateur de l'agent générateur de gaz (également désigné par « agent activateur de gaz ») est un composé qui réagit avec l'agent générateur de gaz par une réaction chimique (préférentiellement une réaction acido-basique) libérant un gaz.

Il s'agit avantageusement d'un acide, d'un sel de polyacide partiellement salifié ou bien d'une solution tampon d'acide faible et de sa base conjuguée, ou d'un mélange de tels composés.

Selon l'invention le tampon acide/base dudit acide peut être tout tampon acide /base de l'acide faible comme par exemple un tampon acide citrique/citrate de sodium ou encore un tampon acide tartrique/tartrate de sodium. De préférence, nous citerons les alpha-hydroxyacides qui sont des acides faibles ayant préférentiellement un pKa compris entre 2 et 6 tel que l'acide citrique, l'acide tartrique, l'acide malique, l'acide lactique mais aussi l'acide phosphorique et l'acide pyrophosphorique et leurs sels éventuellement partiellement salifiés tels que le disodium pyrophosphate ou le sodium dihydrogénophosphate appelé encore phosphate monosodique.

Préférentiellement selon l'invention, l'agent activateur de gaz est choisi parmi un tampon acide tartrique / sel de tartrate (par exemple tartrate de sodium) ; un tampon acide citrique/citrate de sodium seul ; l'acide phosphorique, le phosphate de sodium, le disodium pyrophosphate seuls ou en mélange avec le tampon acide citrique/ citrate de sodium.

Selon un mode de réalisation très préféré, l'agent activateur de gaz est un tampon acide citrique/ citrate de sodium seul ou en mélange avec du phosphate sodique et/ou du disodium pyrophosphate.

Dans des compositions pour peaux sensibles ou pour peaux lésées comme les peaux atteint de psoriasis, la teneur en acide citrique/ citrate de sodium est de préférence inférieure ou égale à 2,4% par rapport au poids total de la composition intermédiaire A, de manière à limiter tout risque de picotement. Afin d'améliorer la tolérance et d'éviter la sensation de picotement, de manière préférée, le tampon acide citrique/citrate de sodium est employé en mélange avec le disodium pyrophosphate ou le sodium dihydrogénophosphate.

Selon l'invention, ledit agent activateur de gaz peut être présent dans la formule intermédiaire A en une quantité pouvant aller de 0,001% à 95% en poids par rapport au poids total de la composition intermédiaire A.

### AGENT GENERATEUR DE GAZ :

Par agent générateur de gaz, on entend tout agent qui possède la propriété de générer par réaction chimique un gaz. On citera à cet égard tout composé qui, lorsqu'il est mélangé à un acide faible, peut former un gaz par une réaction chimique équivalente à la suivante :

NaHCO₃ + RCOOH → RCOONa + H₂O + CO₂

Selon l'invention, le gaz généré à partir de l'agent générateur de gaz présent dans la composition intermédiaire B est de préférence du gaz carbonique ou dioxyde de carbone (CO₂).

Selon l'invention, l'agent générateur de gaz est de préférence choisi parmi le bicarbonate de sodium, le bicarbonate de potassium, le carbonate de sodium, le carbonate de potassium et leurs mélanges.

Préférentiellement selon l'invention, la formule intermédiaire B comprend un agent générateur de gaz carbonique qui de manière particulièrement préférée est du bicarbonate de sodium.

Ledit agent générateur de gaz peut être présent dans la formule intermédiaire B en une quantité allant de 1% à 10%, préférentiellement de 2% à 8% en poids par rapport au poids de la composition intermédiaire B.

Selon l'invention, la formule intermédiaire A peut présenter un pH acide, avantageusement compris entre 1 et 6, et la formule intermédiaire B peut présenter un pH basique, avantageusement compris entre 7 et 12.

Selon l'invention, l'une (ou les) formule(s) intermédiaire(s) comprennent du propionate de clobétasol en quantité allant de 0,0001 à 1% en poids, préférentiellement de 0,001 à 0.5% en poids, encore plus préférentiellement de 0.03 à 0.2% en poids, par rapport au poids de la composition totale.

Dans la présente description, on entend par composition totale ou formule totale la composition du produit sous forme de mousse après le mélange desdites compositions intermédiaires.

De façon préférée, le propionate de clobétasol est contenu dans la composition A formulée à pH acide afin d'optimiser sa stabilité.

La formule intermédiaire A peut se présenter sous toutes les formes galéniques compatibles avec la forme galénique désirée pour la composition finale obtenue par mélange de la formule A avec la formule B. Avantageusement la formule A peut-être un gel, une solution, une suspension, une émulsion (crème, crème sans tensioactif, lotion, lait, crème fluide), de préférence un gel garantissant la stabilité du propionate de clobetasol. Selon un mode de réalisation particulièrement préféré, la formule intermédiaire A est sous forme de gel.

La formule intermédiaire B peut se présenter sous toutes les formes galéniques compatibles avec la forme galénique désirée pour la composition finale obtenue par mélange de la formule B avec la formule A. Avantageusement la formule B peut être un gel, une solution, une suspension, une émulsion (crème, crème sans tensioactif, lotion lait, crème fluide), de préférence un gel, ou une solution.

Selon un mode de réalisation de l'invention, l'une des deux formules intermédiaires (ie,la formule intermédiaire A ou la formule intermédiaire B) se présente sous forme d'un gel. Dans ce mode de réalisation, de préférence l'autre formule intermédiaire n'est pas sous forme de gel.

Chaque formule du kit ou du contenant à plusieurs compartiments tel que défini précédemment, conforme à l'invention comprend un milieu physiologiquement acceptable, véhiculant le ou les composés, et choisi de telle sorte que les composés soient aptes à réagir l'un avec l'autre pour former une composition auto-moussante lors du mélange d'au moins les formules intermédiaires A et B.

Ainsi, le mélange extemporané d'au moins deux formules, par exemple la formule A et la formule B, crée la composition sous forme de mousse selon l'invention.
Lors du mélange des deux formules A et B, l'agent générateur de gaz, tel que le bicarbonate de sodium, peut réagir avec l'agent activateur de gaz tel que l'acide, et donner ainsi notamment le sel correspondant à l'acide, de l'eau et le gaz CO₂. C'est ce gaz, emprisonné dans les bulles de la composition, qui crée la mousse qui caractérise la composition auto-moussante de l'invention.
Ainsi par le mélange d'au moins la formule intermédiaire A et la formule intermédiaire B, on obtient la composition mousse dite composition totale selon l'invention.
Dans la composition obtenue après mélange d'au moins les formules A et B, il peut bien entendu rester de l'agent activateur de gaz et/ou de l'agent générateur de gaz n'ayant pas réagi.

Avantageusement, le kit ou le contenant unique à plusieurs compartiments selon l'invention peut être conçu de telle sorte que lors de la préparation de la composition selon l'invention, les formules intermédiaires A et B peuvent être mélangées en un rapport en poids A/B allant de 0,5 à 2, préférentiellement de 0,5 à 1,5, plus préférentiellement proche de 1 (c'est-à-dire de 0,9 à 1,1), et encore plus préférentiellement de 1. Cela signifie que le kit peut être conçu pour libérer simultanément des doses (en poids) des compositions intermédiaires A et B pouvant être en un rapport en poids allant de 2 doses de B pour 1 dose de A à 2 doses de A pour 1 dose de B, de préférence de 2 doses de B pour 1 dose de A à 3 doses de A pour 2 doses de B. Selon un mode de réalisation préféré de l'invention, le kit est conçu pour libérer simultanément 1 dose en poids de A et 1 dose en poids de B.

Selon l'invention le kit peut se présenter sous toute forme compatible avec d'une part une conservation séparée des formules intermédiaires A et B et d'autre part la capacité à réaliser extemporanément le mélange de A et de B.
Par exemple les formules intermédiaires A et B peuvent être conditionnées dans un boitier avec au moins deux compartiments séparés, chacun contenant les formules A ou B.
Selon un autre aspect, le kit peut se présenter sous la forme d'une seringue à au moins deux corps séparés, chacun muni d'un piston, lesdits deux corps contenant les formules A et B, et étant conçus pour libérer simultanément par exercice d'une force sur le piston les doses désirées des formules A et B.

L'invention concerne également un procédé de préparation d'une composition selon l'invention, caractérisé en ce que pour obtenir la composition sous forme de mousse, on mélange de façon extemporanée au moins une dose de formule intermédiaire A et une dose de formule intermédiaire B du kit telles que définies plus haut, dans des proportions relatives en poids A/B pouvant aller de 0,5 à 2, préférentiellement de 0,5 à 1,5 et plus préférentiellement de 1.

Afin d'obtenir une mousse (composition finale) optimale, les inventeurs ont cherché expérimentalement les teneurs optimales en agent générateur de gaz (de préférence le bicarbonate de sodium) et en agent activateur de gaz (de préférence l'acide citrique et/ou le disodium pyrophosphate et/ou le sodium dihydrogénophosphate).

Ainsi, il a été déterminé expérimentalement que lorsque l'agent activateur de gaz est l'acide citrique, le ratio pondéral acide citrique/bicarbonate de sodium dans la composition totale est compris entre 0,1 et 2 préférentiellement entre 0,5 à 1 et de façon préférée égal à 0,7.

De la même façon, il a été déterminé que lorsque l'agent activateur de gaz est le disodium pyrophosphate, le ratio pondéral disodium pyrophosphate/bicarbonate de sodium dans la composition totale est compris entre 0,5 et 5 préférentiellement entre 1 et 3 et de façon préférée est égal à 2,4.

De la même façon, il a été déterminé que lorsque l'agent activateur de gaz est le sodium dihydrogénophosphate, le ratio pondéral sodium dihydrogénophosphate mono hydrate/bicarbonate de sodium dans la composition totale est compris entre 0,5 et 5 préférentiellement entre 1 et 3 et de façon préférée est égal à 2.

L'exemplification des ratios bicarbonate de sodium/acide citrique, bicarbonate de sodium/pyrophosphate de sodium, bicarbonate de sodium/sodium hydrogénophosphate est décrit dans l'exemple 4.

De façon surprenante, l'association acide citrique/citrate de sodium, disodium pyrophosphate et un système gélifiant compatible avec la forme galénique a permis d'obtenir une formule aux propriétés physico-chimiques très stables et dans laquelle le propionate de clobétasol est particulièrement stable ne générant aucune sensation désagréable sur la peau et permettant la libération de gaz et ainsi la création de mousse.

L'exemple 2B ci-dessous montre que les compositions selon la présente invention présentent une excellente stabilité tant physique que chimique.

Une composition est considérée stable physiquement lorsque ses caractéristiques organoleptiques, son pH, sa viscosité et l'homogénéité du propionate de clobetasol n'évoluent pas avec le temps dans différentes conditions de températures : température ambiante (RT, ou TA), 30°C et 40°C.
Selon l'invention, la température ambiante correspond à une température allant de 15°C à 25°C.
Une composition est considérée stable chimiquement lorsque la teneur en principe actif qu'elle contient n'évolue pas avec le temps dans les différentes conditions de températures (RT, 30°C et 40°C).

Selon l'invention, la composition est considérée stable quand la teneur en principe actif (exprimée en poids par rapport au poids de la formule intermédiaire) mesurée par toute technique connue et en particulier par HPLC, est incluse dans les spécifications allant de 90% à 110%.

La composition selon l'invention peut comprendre en outre un ou plusieurs agents choisis parmi les agents dispersants, les agents solubilisants, les agents stabilisants, les agents conservateurs, les corps gras, les agents épaississants, les colorants, les parfums, les tensioactifs, les agents gélifiants, les agents complexants, les agents neutralisants, les agents émulsionnants moussants, les agents émulsionnants non moussants, les charges, les agents séquestrants, les agents réducteurs, les masques d'odeur, les agents plastifiants, les agents adoucissants, les agents hydratants, les pigments, les argiles, les charges minérales, les colloïdes minéraux, les polymères, les protéines, les agents nacrants, les cires, les huiles comme par exemple les paraffines, les silicones, des acides gras, des esters solides d'alcool gras ou d'acides gras, des gommes, les agents mouillants.

Bien entendu, l'homme du métier veillera à choisir ce ou ces éventuels adjuvants additionnels et/ou leur quantité de manière telle que les propriétés de (ou des) actif(s) pouvant être ajoutés à la composition selon l'invention ne soient pas, ou substantiellement pas, altérées par l'adjonction envisagée.

Des colorants hydrosolubles tels que le FD&C Blue 1 (de formule brute C₃₇H₃₄N₂Na₂O₉S₃) et des colorants liposolubles tel que le Rouge Soudan III ou le Red Nil présentent l'avantage de colorer un des intermédiaires de formulation. Cette coloration permet le contrôle du bon mélange des deux intermédiaires de formulation et de mettre en valeur la formation de la mousse.

### AGENTS GELIFIANTS DE LA FORMULE INTERMEDIAIRE CONTENANT L'ACTIVATEUR DE GAZ

La composition intermédiaire A contenant au moins un agent activateur de gaz contient au moins un agent gélifiant et/ou agent de suspension tel que défini dans la revendication 1.

La formulation A peut contenir des fortes quantités d'acide et d'électrolytes.

A titre d'exemple de gélifiants et/ou agents de suspension résistants à la fois aux électrolytes et aux pH acides pouvant entrer dans les compositions A selon l'invention, on peut citer les mélanges prêt à l'emploi tels que le Polyacrylate-13 & Polyisobutene & Polysorbate 20 vendus par Seppic sous le nom Sepiplus 400®, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M® premium par la société Dow Chemical ou l'hydroxyéthylcellulose, en particulier, le produit vendu sous le nom de Natrosol HHX 250® par la société Aqualon, la famille des aluminium magnésium silicates tel que le Veegum K®, Veegum Plus® ou le Veegum Ultra® vendu par la société Vanderbilt, la bentonite vendue sous le nom de Polargel® HV, la famille des amidons modifiés tels que l'amidon de pomme de terre modifié vendu sous le nom de Structure Solanace®, la famille des carraghénanes en particulier réparties sous quatre grandes familles : κ, λ, β, ω tel que les Viscarin® et les Gelcarin® commercialisés par la société IMCD. Ou encore la Polyvinyl Alcohol connue aussi sous l'abréviation PVA revendu par Merck sous le nom POLYVINYL ALCOHOL 40-88®. De façon préférée, le Vegum K® et le Xantural 180® seront utilisés en association.

L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire A.

### AGENTS GELIFIANTS DE LA FORMULE INTERMEDIAIRE CONTENANT LE GENERATEUR DE GAZ

La composition intermédiaire B contenant au moins un agent générateur de gaz contient au moins un agent gélifiant et/ou agent de suspension tel que défini dans la revendication 1.

A titre d'exemple de gélifiants et/ou agents de suspension et/ou gélifiants résistants à la fois aux électrolytes et aux pH basiques pouvant entrer dans les compositions intermédiaires B selon l'invention, on peut citer les polymères d'acide acryliques comme l'Acrylates/C10-30 Alkyl Acrylate Crosspolymer tel que les carbomers dits non sensibles aux électrolytes, vendus sous le nom d'Ultrez 20®, d'Ultrez 10®, de Carbopol 1382® ou de Carbopol ETD2020NF®, AQUA SF1® vendus par la Société Lubrizol, le mélange Ammonium Acrylate / Acrylamide Copolymer & Polyisobutene & Polysorbate 20 vendu par Seppic sous le nom Sepiplus 265®, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® ou Jaguard S revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M premium® par la société Dow Chemical ou l'hydroxyéthylcellulose, en particulier, le produit vendu sous le nom de Natrosol HHX 250® par la société Aqualon, la famille des aluminium magnésium silicates tel que le Veegum K®, Veegum Plus® ou le Veegum Ultra® vendu par la société Vanderbilt, la bentonite vendue sous le nom de Polargel® HV, la famille des amidons modifiés tels que l'amidon de pomme de terre modifié vendu sous le nom de Structure Solanace® ou de Farine de Tapioca connue sous le nom de Naviance Tapioca P® revendu par Akzo Novel ou de la famille des carraghénanes en particulier réparties sous quatre grandes familles : κ, λ, β, ω tel que les Viscarin® et les Gelcarin® commercialisés par la société IMCD.

L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire B.

### AGENTS HUMECTANTS

Parmi les agents humectants et/ou émollients qui ont pour rôle d'hydrater la peau et de faciliter l'application de la formulation, on utilise optionnellement, sans que cette liste soit limitative, des composés tels qu'un polyol miscible à l'eau à la température ambiante (25°C) notamment choisi parmi les polyols ayant notamment de 2 à 20 atomes de carbones, de préférence ayant de 2 à 10 atomes de carbone, et préférentiellement ayant de 2 à 6 atomes de carbones, tels que le glycérol, les dérivés de glycol tel que le propylène glycol, le butylène glycol, le pentylène glycol, l'hexylène glycol, le dipropylène glycol, le diéthylène glycol et leurs mélanges mais aussi les sucres (à titre d'exemple le glucose, le lactose), les polyethylene glycol (PEG) (à titre d'exemple le Lutrol E400), l'urée, les acides aminés (à titre d'exemple la sérine, la citrulline, l'arginine, l'asparagine, l'alanine).
A titre d'agent humectant et/ou émollient préféré, on peut citer la glycérine et le propylene glycol.

Les agents humectants peuvent être utilisés, seuls ou en association, aux concentrations préférentielles allant de 0,001% à 30 % et, plus préférentiellement, allant de 0.01% à 10% en poids par rapport au poids de la formule totale.

### SOLVANT ET AGENT PROPENETRANT

La composition selon l'invention peut comprendre un ou plusieurs solvants et/ou agents propénétrants, qui permettent de faciliter la pénétration des principes actifs, et de dissoudre le principe actif présent dans la composition selon l'invention. Plus particulièrement, il est choisi parmi les alcools volatiles en C1-C4, comme l'éthanol ou l'isopropanol, ou encore parmi les éthers de glycol, tels que l'éthoxydiglycol vendu par la Gattefossé sous le nom Transcutol HP. Il peut être compris dans la formule A à une teneur allant de 0.1% à 10% et plus préférentiellement entre 0.5 et 2% en poids par rapport au poids de la formule intermédiaire A.

### AGENTS CHELATANTS

Parmi les agents chélatants, on peut citer à titre d'exemples non limitatifs l'acide éthylène diamine tétra-acétique (EDTA), l'acide diéthylène triamine penta-acétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA).

A titre d'agent chélatant préféré, on peut citer l'acide éthylène diamine tétra-acétique (EDTA) vendu notamment sous le nom Titriplex III®. Il peut être utilisé aux concentrations préférentielles allant de 0,001% à 1 % et, plus préférentiellement de 0.05% à 0.1% en poids par rapport au poids de la formule totale.

### EXCIPIENTS AUX PROPRIETES SPECIFIQUES

La composition selon l'invention peut contenir un ou plusieurs actifs cosmétiques comme par exemple titre non limitatif l'allantoine aux propriétés anti irritantes, le dipotassium dlycyrrhizate pour ces propriétés anti inflammatoires, l'acide salicylique et les goudrons de houille aux propriétés keratolytiques, la capsaïcine qui atténue les démangeaisons ou encore le alpha bisabol cicatrisant ou encore des agents conditionneurs du cheveu tel que les Polyquaterniums.

### CHARGES ET PARTICULES

Des charges et/ou des particules peuvent être utilisées pour stabiliser et booster la mousse. Certaines d'entre elles ont la propriété particulière de se placer à l'interface eau/air et ainsi stabiliser cette interface. Comme charges, on peut citer le talc, les oxydes de métaux tel que l'oxyde de zinc, le dioxyde de titane TiO2 T2000 vendu par la société Merck sous le nom Eusolex® T-2000, les argiles tel que les laponites®, bentones® ou les bentonites® mais aussi les éthers de cellulose tel que le Methocel K100 LV® commercialisé par la société DOW, les silices telles que l'Aerosil® R972 vendue par la société EVONIK ou la SILICE HDK® H13L vendue par WACKER. Elles peuvent être utilisées aux concentrations allant de 0.01% à 10% en poids par rapport au poids de la formule totale.

### AGENT CONSERVATEUR :

On peut citer à titre d'exemple de conservateurs, le chlorure de benzalkonium, le bronopol, la chlorhexidine, le chlorocrésol et ses dérivés, l'alcool éthylique, le phénoxyéthanol, le sorbate de potassium, la diazolidinylurée, l'alcool benzylique, les parabens, le benzoate de sodium ou leurs mélanges.

Au titre de système conservateur préféré on peut citer l'association phenoxyethanol et pentylene glycol ou le benzoate de sodium.

### TENSIOACTIFS MOUSSANTS DE LA COMPOSITION

La composition intermédiaire A peut contenir une petite quantité de tensio-actifs moussant, avantageusement compatibles avec le propionate de clobétasol.

Comme exemples de tensioactifs utilisables, on peut citer :les tensio-actifs anioniques de la famille des sulfonate tel que Sodium C14-C16 Olefin Sulfonate, de la famille des glycinates tel que le Sodium Cocoyl Glycinate revendu par Clariant sous le nom Hostapon SG, de la famille des isethionates tels que Sodium Cocoyl Isethionate revendu par Clariant sous le nom Hostapon SCI85 G, Sodium Lauroyl Methyl Isethionate revendu par Innospec sous le nom Iselux LG, de la famille des sulfates tel que Zinc Coceth Sulfate revendu par Zschimmer & Schwarz sous le nom Zetesol Zn ou le Sodium Laureth sulfate revendu sous le nom Texapon N70,de la famille des sulfosuccinates tel que Disodium PEG-5 Laurylcitrate Sulfosuccinate revendu par Evonik sous le nom Rewopol SB C55, le Disodium PEG-12 Dimethicone Sulfosuccinate vendu par Rodhia sous le nom de Mackanate ultra Si, le Sodium Cocoamphoacetate ou Disodium Cocoamphodiacetate vendu par Evonik sous le nom Rewoteric AMC et Rewoteric AM2CNM, le sodium cocoyl glutamate rendu sous le nom Protelan AGL95 par Zschimmer & Schwarz, le sodium capryolyl glutamate vendu sous le nom Protelan AG8 par Zschimmer & Schwarz ou encore le sodium lauroyl saccosinate vendu par Zschimmer & Schwarz sous le nom Protelan LS911.

On peut aussi employer des tensio-actifs non ioniques tels que Decyl Glucoside revendu par Cognis sous le nom Plantacare 2000 Up, le Glyceryl Monolaurate revendu par Rossow sous le nom POEM DL 100, le Sucrose Laurate vendu par Sisterna sous le nom Sisterna L70-C.

On peut encore employer des tensio-actifs amphotères tels que ceux de la famille des bétaïnes tel que la cocobétaïne revendu sous le nom dehyton AB30 ou la cocamidopropyl bétaïne revendu sous le nom Tego betaine F50.

Ces tensio-actifs peuvent être utilisés seuls ou en association. La teneur totale de ces tensio-actifs est de préférence inférieure ou égale à 2,5% en poids, et plus préférentiellement inférieure ou égale à 1% en poids, par rapport au poids de la composition totale.

Selon un mode de réalisation, la composition selon l'invention ne contient pas de tensioactif moussant.

Les exemples suivants illustrent l'invention sans en restreindre la portée.

### EXEMPLES :

### EXEMPLE 1 : Exemples de formules

### Exemples de formules A : compositions intermédiaires contenant l'agent activateur de gaz, formulées à pH acide

Les formules intermédiaires A ont été préparées suivant le procédé suivant :
Etape 1 : A une température supérieure à 60°C, ajouter sous agitation les gélifiants puis le ou les agents activateurs du générateur de gaz à la phase d'eau principale.
Etape 2 : Parallèlement réaliser la phase active en solubilisant le propionate de clobétasol dans l'agent solubilisant/pro-pénétrant
Etape 3 : A une température inférieure à 30°C ajouter la phase active à la phase principale.
Etape 4 : Ajouter les additifs ainsi que les conservateurs, les actifs cosmétiques, les chélatants.

Dans les exemples de formules ci-dessous, les quantités sont exprimées par rapport au poids de la formule intermédiaire et non par rapport au poids de la formule totale.

### Exemple A1

| INCI Name | % |
|---|---|
| WATER | QSP 100 |
| DISODIUM EDTA | 0.1 |
| MAGNESIUM ALUMINIUM SILICATE | 2.5 |
| XANTHAN GUM | 0.7 |
| CITRIC ACID | 1.4 |
| SODIUM CITRATE | 1 |
| SODIUM PYROPHOSPHATE | 7.2 |
| SODIUM LAURETH SULFATE | 3.6 |
| COCO BETAINE | 1.3 |
| PROPYLENE GLYCOL | 4 |
| ETHOXYDIGLYCOL | 1.2 |
| CLOBETASOL PROPIONATE | 0.1 |
| SODIUM BENZOATE | 0.2 |

### Exemple A2

| INCI Name | % |
|---|---|
| WATER | QSP 100 |
| DISODIUM EDTA | 0.1 |
| MAGNESIUM ALUMINIUM SILICATE | 2.5 |
| XANTHAN GUM | 0.7 |
| CITRIC ACID | 3.5 |
| SODIUM CITRATE | 2.7 |
| SODIUM LAURETH SULFATE | 3.6 |
| COCO BETAINE | 1.3 |
| PROPYLENE GLYCOL | 4 |
| ETHOXYDIGLYCOL | 1.2 |
| CLOBETASOL PROPIONATE | 0.1 |
| SODIUM BENZOATE | 0.2 |

### Exemple A3

| INCI Name | % |
|---|---|
| WATER | QSP 100 |
| DISODIUM EDTA | 0.1 |
| MAGNESIUM ALUMINIUM SILICATE | 2.5 |
| XANTHAN GUM | 0.7 |
| CITRIC ACID | 3.5 |
| SODIUM CITRATE | 2.7 |
| PROPYLENE GLYCOL | 4 |
| ETHOXYDIGLYCOL | 3 |
| CLOBETASOL PROPIONATE | 0.1 |
| SODIUM BENZOATE | 0.2 |

### Exemple A4

| INCI Name | % |
|---|---|
| WATER | QSP 100 |
| DISODIUM EDTA | 0.1 |
| MAGNESIUM ALUMINIUM SILICATE | 2.5 |
| GUARD GUM | 0.4 |
| HYDROXYETHYL CELLULOSE | 0.5 |
| CITRIC ACID | 3.5 |
| SODIUM CITRATE | 2.7 |
| SODIUM LAURETH SULFATE | 3.6 |
| COCO BETAINE | 1.3 |
| PROPYLENE GLYCOL | 4 |
| ETHOXYDIGLYCOL | 1.2 |
| CLOBETASOL PROPIONATE | 0.1 |
| SODIUM BENZOATE | 0.2 |

### Exemple A7

| INCI Name | % |
|---|---|
| WATER | QSP 100 |
| DISODIUM EDTA | 0.1 |
| XANTHAN GUM | 0,7 |
| MAGNESIUM ALUMINIUM SILICATE | 2.5 |
| SODIUM BENZOATE | 0.2 |
| POLYVINYL ALCOHOL | 2 |
| DISODIUM PYROPHOSPHATE | 7.2 |
| PHENOXYETHANOL | 0.5 |
| CITRIC ACID | 1.4 |
| SODIUM CITRATE | 1 |
| POLOXAMER 124 | 0,2 |
| CLOBETASOL PROPIONATE | 0.1 |
| PROPYLENE GLYCOL | 4,0 |
| FD&C BLUE 1 | 0.0009 |

### Exemples de formules B : compositions intermédiaires contenant l'agent générateur de gaz

Les formules intermédiaires sont formulées suivant le procédé suivant :
Etape 1' : A une température supérieure à 60°c, Ajouter sous agitation les gélifiants à la phase d'eau principale.
Etape 2' : Ajouter les agents nettoyants ou moussants ainsi que les additifs tels que les conservateurs à une température adaptée.
Etape 3' : Neutraliser le mélange
Etape 4' : A une température inférieure à 40°C ajouter le bicarbonate de sodium.

Dans un mode particulier, une phase grasse (contenant les huiles, les cires, et les tensio-actifs) peut être chauffée à une température supérieure à 60°C et incorporer à la phase principale après l'étape 1'.

### Exemple B1

| INCI Name | % |
|---|---|
| WATER | QSP100 |
| MAGNESIUM ALUMINIUM SILICATE | 2.5 |
| XANTHAN GUM | 0.5 |
| DISODIUM EDTA | 0.1 |
| DIPOTASSIUM GLYCYRRHIZATE | 0.5 |
| ZINC GLUCONATE | 0.4 |
| SODIUM C14-16 OLEFIN SULFONATE | 2 |
| SODIUM HYDROXYDE | 0.1 |
| SODIUM HYDROGENO CARBONATE | 5 |
| PHENOXYETHANOL | 1 |
| PENTYLENE GLYCOL | 5 |

### Exemple B2

| INCI Name | % |
|---|---|
| WATER | QSP100 |
| MAGNESIUM ALUMINIUM SILICATE | 2.5 |
| XANTHAN GUM | 0.6 |
| DISODIUM EDTA | 0.1 |
| SODIUM HYDROXYDE | 0.1 |
| SODIUM HYDROGENO CARBONATE | 3 |
| PHENOXYETHANOL | 1 |

### Exemple B3

| INCI Name | % |
|---|---|
| WATER | QSP100 |
| MAGNESIUM ALUMINIUM SILICATE | 2.5 |
| XANTHAN GUM | 0.5 |
| DISODIUM EDTA | 0.1 |
| DIPOTASSIUM GLYCYRRHIZATE | 0.5 |
| ZINC GLUCONATE | 0.4 |
| SODIUM C14-16 OLEFIN SULFONATE | 2 |
| SODIUM HYDROXYDE | 0.1 |
| SODIUM HYDROGENO CARBONATE | 3 |
| PHENOXYETHANOL | 1 |
| PENTYLENE GLYCOL | 5 |

Le tableau ci-dessous représente les mélanges dans un ratio pondéral 1:1 des compositions intermédiaires A et B décrites ci-avant. Une croix à l'intersection de deux intermédiaires de formulation indique que le mélange a été testé et a généré une mousse aux propriétés recherchées.

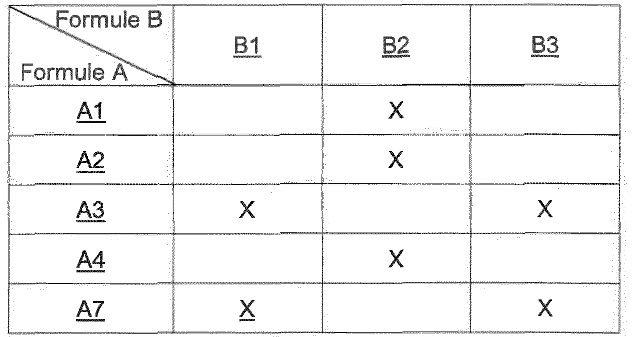

### EXEMPLE 2A : densité de mousse

A partir des exemples de formules décrites dans l'exemple 1, des mesures de densité de mousses ont été réalisées au moment de la mise en contact des 2 formules intermédiaires A et B (T0) puis lorsque la réaction chimique générée par la mise en contact des deux compositions est terminée. Ces études ont montré que la composition finale se présente sous la forme d'une mousse présentant une densité appropriée
La mesure de la densité de la mousse montre que le volume généré par l'un des mélanges proposés dans le tableau ci-dessus a été augmenté d'un facteur 3 à 5

### EXEMPLE 2B : stabilité

Le tableau I ci-dessous rassemble les données de stabilité physique de la formule intermédiaire A1 décrite dans l'exemple 1, contenant du propionate de clobétasol.

| exemple A1 | T0 | | T1Mois | T2Mois | T3Mois |
|---|---|---|---|---|---|
| pH | 4.24 | 25°C | 4.23 | 4.23 | 4.25 |
| | | 40°C | 4.18 | 4.14 | 4.28 |
| Viscosité cP Brookfield RV DVII Aig5 vit20 | 10160 | 25°C | 12020 | 12530 | 10700 |
| | | 40°C | 11450 | 10980 | 13000 |

Les tableaux IIa et IIb ci-dessous détaillent les données de stabilité chimique du propionate de clobétasol dans les formules intermédiaires A1 et A2 décrites dans l'exemple 1.

| exemple A1 | T0 | | T1Mois | T2Mois | T3Mois |
|---|---|---|---|---|---|
| %P/P Propionate de clobétasol (HPLC) | 101.4 | TA | 99,4 | 99,1 | 99,0 |
| | | 40°C | 99,0 | 103,7 | 102,9 |

| exemple A2 | T0 | | T1 Mois | T2Mois | T3Mois |
|---|---|---|---|---|---|
| %P/P Propionate de clobétasol (HPLC) | 101.4 | TA | 99,5 | 99,6 | 100,4 |
| | | 40°C | 99,9 | 99,7 | 99,7 |

### EXEMPLE 3 : Etude comparative de mesure de l'irritation

### Protocole d'étude.

L'étude est réalisée selon le protocole TG439 OCDE en vigueur pour le temps d'application court (temps de contact RHE/produit 15min). Ce protocole est adapté pour un temps d'application long (temps de contact RHE/produit 18h).
L'objectif de cette étude est d'évaluer la tolérance des supports des formules complètes et intermédiaire sur épidermes humains reconstruits (RHE, modèle Episkin) au travers de :
- l'évaluation de la réduction du MTT (viabilité cellulaire)
- la mesure de la libération d'IL-1 alpha (marqueur d'irritation)

Les formules testées sont :
- Une composition intermédiaire de formulation acide : exemple A7 placebo (c'est à dire, ne contenant pas de proprionate de clobétasol) non colorée (c'est-à-dire, ne contenant pas de colorant)
- Une composition intermédiaire de formulation basique: exemple B1
- La formule complète 1 composée du mélange : A7 placebo + B1 (dans un ratio 50/50 en poids)
- Une référence commerciale sous forme gel nettoyant

| Mélange testé | Exposition courte | Exposition longue | Conclusion |
|---|---|---|---|
| | Viabilité (%) | Viabilité (%) | Potentiel irritant |
| B1 | 92.5 | 59.6 | Non irritant |
| A7 placebo | 86.0 | 84.5 | Non irritant |
| Formule complète 1 | 93.2 | 86.8 | Non irritant |
| Ref commerciale | 76.6 | 6.7 | Potentiellement irritant |

| Test item | Exposition courte | Exposition longue | Conclusion |
|---|---|---|---|
| | IL-1a vs contrôle | IL-1a vs contrôle | Potentiel irritant |
| B1 | 5.5 | 30.6 | Non irritant |
| A7 placebo | 2.2 | 2.3 | Non irritant |
| Formule complète 1 | 4.5 | 11.6 | Non irritant |
| Ref commerciale | 6.2 | 113.9 | Potentiellement irritant |

Les mesures du MTT selon le protocole OCDE en Vigueur indique que la « Formule complète » rincée testée est non irritantes alors que la « Ref commerciale » est Potentiellement irritante.
De plus, le dosage d'IL-1a après application à des temps d'exposition longs et courts des formules complètes montre un taux de marqueurs d'irritation beaucoup plus bas sur les formules mousses qu'après application de la référence commerciale.

### EXEMPLE 4 :

Il a été établi de façon empirique la teneur idéale en acide citrique, pyrophosphate de sodium et sodium dihydrogénophosphate monohydrate pour réagir avec 5% de bicarbonate de sodium. Les valeurs sont exprimées en pourcentage poids/poids par rapport au poids de chacune des deux formules intermédiaires

| | Ratio 1 | Ratio2 | Ratio3 |
|---|---|---|---|
| Bicarbonate de Sodium | 5% | 5% | 5% |
| Acide citrique | 3.5% | - | - |
| Disodium pyrophosphate | - | 12 | - |
| Sodium Dihydrogénophosphate monohydrate | | - | 7.2% |

Afin que le pH de la formule contenant l'activateur de gaz présente une compatibilité optimale avec la peau, nous avons ajouté du citrate de sodium afin de créer un tampon acide citrique/citrate de sodium.
Une partie du tampon acide citrique/citrate de sodium peut avantageusement être substituée par du disodium pyrophosphate et vice versa, suivant les teneurs citées à titre d'exemple dans le tableau I ci-dessous :

**Tableau III : Les valeurs sont exprimées en pourcentage poids/poids par rapport au poids de chacune des deux formules intermédiaires**

| | E 1 | E2 | E 3 | E 4 | E 5 | E 6 | E 7 |
|---|---|---|---|---|---|---|---|
| Bicarbonate de sodium | 5% | 5% | 5% | 5% | 3% | 3% | 3% |
| Acide citrique | 3.5% | 1.75% | 1.4% | 0 | 2.1% | 1.05% | 0 |
| Citrate de Na | 2.7% | 1.3% | 1% | 0 | 1.6% | 1.15% | 0 |
| Disodium pyrophosphate | 0 | 6% | 7.2% | 12% | 0 | 3.6% | 7.2% |

Une partie du tampon l'acide citrique/citrate de sodium peut avantageusement être substituée par du sodium dihydrogénophosphate mono hydrate et vice versa suivant les teneurs citées à titre d'exemple dans le tableau IV ci-dessous :

**Tableau IV : Les valeurs sont exprimées en pourcentage poids/poids par rapport au poids de chacune des deux formules intermédiaires.**

| | E 1 | E8 | E9 |
|---|---|---|---|
| Bicarbonate de Sodium | 5% | 5% | 5% |
| Acide citrique | 3.5% | 1.5% | 0 |
| Citrate de Na | 2.7% | 0.5% | 0 |
| Sodium dihydrogénophosphate monohydrate | 0 | 6.2% | 10% |

Dans un mode particulier, il a été déterminé que lorsque la quantité d'acide citrique est supérieure ou également à 1,4, la quantité de mousse est optimale lorsque le disodium pyrophosphate est présent dans la composition selon l'équation suivante :
- [C]=2.4[B]-2.4[A]/0.7
Lorsque :
[C] = teneur en poids en disodium pyrophosphate dans la composition intermédiaire A
[A] = teneur en poids en acide citrique monohydrate dans la composition intermédiaire A
[B] = teneur en poids en bicarbonate de sodium dans la composition intermédiaire B

L'équation ci-dessus permet ainsi de calculer les teneurs optimales entre le bicarbonate de sodium l'acide citrique et le pyrophosphate de sodium.

## Revendications

1. Composition auto-moussante contenant du propionate de clobétasol destinée à une application topique rincée, comprenant:
- au moins une composition intermédiaire B comprenant un agent générateur de gaz et au moins un agent gélifiant et/ou agent de suspension choisi parmi les polymères d'acide acrylique, les polysaccharides, la famille des aluminium magnésium silicates, la famille des amidons modifiés et la famille des carraghénanes,
- au moins une composition intermédiaire A comprenant un agent activateur de l'agent générateur de gaz et au moins un agent gélifiant et/ou agent de suspension choisi parmi les polysaccharides, la famille des aluminium magnésium silicates, la famille des amidons modifiés, la famille des carraghénanes et le PVA. et
- du propionate de clobétasol contenu dans l'une au moins desdites compositions intermédiaires A et B.

2. Composition selon la revendication précédente, **caractérisée en ce que** le propionate de clobétasol est contenu dans la composition intermédiaire A.

3. Composition selon l'une quelconque des revendications précédentes, **caractérisée en ce qu'**elle ne comprend pas de tensioactifs moussants, ou qu'elle contient de tels tensioactifs en une teneur inférieure ou égale à 2,5% en poids, de préférence inférieure ou égale à 1% en poids, par rapport au poids de la composition totale.

4. Composition selon l'une quelconque des revendications précédentes, **caractérisée en ce que** l'agent générateur de gaz est choisi parmi le bicarbonate de sodium, le bicarbonate de potassium, le carbonate de sodium, le carbonate de potassium et leurs mélanges, et de préférence l'agent générateur de gaz est le bicarbonate de sodium.

5. Composition selon l'une quelconque des revendications précédentes, **caractérisée en ce que** l'agent générateur de gaz est présent dans la composition intermédiaire B en une quantité allant de 1% à 10%, préférentiellement de 2% à 8% en poids, par rapport au poids de la composition intermédiaire B.

6. Composition selon l'une quelconque des revendications précédentes, **caractérisée en ce que** l'agent activateur de l'agent générateur de gaz est choisi parmi un acide, un sel de polyacide partiellement salifié, une solution tampon d'acide faible et de sa base conjuguée, et les mélanges de ces composés.

7. Composition selon la revendication précédente, **caractérisée en ce que** l'agent activateur de l'agent générateur de gaz est choisi parmi l'acide citrique, l'acide tartrique, l'acide malique, l'acide lactique, l'acide phosphorique et l'acide pyrophosphorique, et les sels de ces acides, et plus préférentiellement ledit agent activateur est choisi parmi :
- un tampon acide citrique/citrate de sodium seul ;
- l'acide phosphorique, le phosphate de sodium, le disodium pyrophosphate seuls ou en mélange avec un tampon acide citrique/ citrate de sodium.

8. Composition selon la revendication précédente, **caractérisée en ce que** l'agent activateur de l'agent générateur de gaz est un tampon acide citrique/ citrate de sodium seul ou en mélange avec du phosphate sodique et/ou du disodium pyrophosphate.

9. Composition sous forme de mousse, **caractérisée en ce qu'**elle résulte du mélange desdites compositions intermédiaires A et B selon l'une quelconque des revendications précédentes.

10. Composition selon l'une quelconque des revendications précédentes, **caractérisée en ce qu'**elle contient en outre un ou plusieurs actifs choisi parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

11. Médicament destiné à une application topique sur la peau, comprenant une composition telle que définie dans l'une quelconque des revendications 1 à 10.

12. Composition selon l'une quelconque des revendications 1 à 10, pour son utilisation dans le traitement du psoriasis.

13. Kit ou contenant unique à plusieurs compartiments destiné à une application topique rincée comprenant de manière séparée au moins deux compositions intermédiaires:
- une composition intermédiaire A comprenant au moins un agent activateur de l'agent générateur de gaz, telle que définie dans l'une quelconque des revendications 1 à 10; et
- une composition intermédiaire B comprenant au moins un agent générateur de gaz telle que définie dans l'une quelconque des revendications 1 à 10 ;
- du propionate de clobétasol étant contenu dans l'une au moins desdites compositions intermédiaires A et B.

14. Kit ou contenant unique à plusieurs compartiments selon la revendication 13, **caractérisé en ce qu'**il est conçu pour mélanger les compositions intermédiaires A et B en un rapport en poids A/B allant de 0,5 à 2, préférentiellement de 0,5 à 1,5, plus préférentiellement de 0,9 à 1,1, et encore plus préférentiellement de 1.

15. Procédé de préparation d'une composition sous forme de mousse destinée à une application topique non rincée contenant du propionate de clobétasol, dans lequel on mélange une composition intermédiaire A telle que définie dans l'une quelconque des revendications 1 à 10, avec une composition intermédiaire B telle que définie dans l'une quelconque des revendications 1 à 10, dans des proportions relatives en poids A/B allant de 0,5 à 2, préférentiellement de 0,5 à 1,5 et plus préférentiellement de 1.

## Patentansprüche

1. Selbstschäumende Zusammensetzung, enthaltend Clobetasolpropionat, zur topischen Anwendung mit Spülen, umfassend:
- mindestens eine Zwischenzusammensetzung B, umfassend ein Gaserzeugungsmittel und mindestens ein Geliermittel und/oder Suspendiermittel, ausgewählt aus Acrylsäurepolymeren, Polysacchariden, der Familie der Aluminiummagnesiumsilikate, der Familie der modifizierten Stärken und der Familie der Carrageene,
- mindestens eine Zwischenzusammensetzung A, umfassend ein Aktivierungsmittel für das Gaserzeugungsmittel und mindestens ein Geliermittel und/oder Suspendiermittel, ausgewählt aus Polysacchariden, der Familie der Aluminiummagnesiumsilikate, der Familie der modifizierten Stärken, der Familie der Carrageene und PVA, und
- Clobetasolpropionat, das in mindestens einer der Zwischenzusammensetzungen A und B enthalten ist.

2. Zusammensetzung nach dem vorhergehenden Anspruch, **dadurch gekennzeichnet, dass** das Clobetasolpropionat in der Zwischenzusammensetzung A enthalten ist.

3. Zusammensetzung nach einem der vorhergehenden Ansprüche, **dadurch gekennzeichnet, dass** sie keine schäumenden Tenside umfasst oder dass sie solche Tenside in einem Gehalt von weniger als oder gleich 2,5 Gew.-%, vorzugsweise weniger als oder gleich 1 Gew.-%, bezogen auf das Gewicht der Gesamtzusammensetzung, enthält.

4. Zusammensetzung nach einem der vorhergehenden Ansprüche, **dadurch gekennzeichnet, dass** das Gaserzeugungsmittel aus Natriumbicarbonat, Kaliumbicarbonat, Natriumcarbonat, Kaliumcarbonat und ihren Gemischen ausgewählt ist und vorzugsweise das Gaserzeugungsmittel Natriumbicarbonat ist.

5. Zusammensetzung nach einem der vorhergehenden Ansprüche, **dadurch gekennzeichnet, dass** das Gaserzeugungsmittel in der Zwischenzusammensetzung B in einer Menge von 1 bis 10 Gew.-%, bevorzugt von 2 bis 8 Gew.-%, bezogen auf das Gewicht der Zwischenzusammensetzung B, vorliegt.

6. Zusammensetzung nach einem der vorhergehenden Ansprüche, **dadurch gekennzeichnet, dass** das Aktivierungsmittel für das Gaserzeugungsmittel aus einer Säure, einem partiell versalzten Polysäuresalz, einer Pufferlösung einer schwachen Säure und ihrer konjugierten Base und den Gemischen dieser Verbindungen ausgewählt ist.

7. Zusammensetzung nach dem vorhergehenden Anspruch, **dadurch gekennzeichnet, dass** das Aktivierungsmittel für das Gaserzeugungsmittel aus Zitronensäure, Weinsäure, Äpfelsäure, Milchsäure, Phosphorsäure und Pyrophosphorsäure und den Salzen dieser Säuren ausgewählt ist und stärker bevorzugt das Aktivierungsmittel ausgewählt ist aus:
- einem Zitronensäure/Natriumcitrat-Puffer allein,
- Phosphorsäure, Natriumphosphat, Dinatriumpyrophosphat allein oder im Gemisch mit einem Zitronensäure/Natriumcitrat-Puffer.

8. Zusammensetzung nach dem vorhergehenden Anspruch, **dadurch gekennzeichnet, dass** das Aktivierungsmittel für das Gaserzeugungsmittel ein Zitronensäure/Natriumcitrat-Puffer allein oder im Gemisch mit Natriumphosphat und/oder Dinatriumpyrophosphat ist.

9. Zusammensetzung in Schaumform, **dadurch gekennzeichnet, dass** sie aus dem Mischen der Zwischenzusammensetzungen A und B nach einem der vorhergehenden Ansprüche resultiert.

10. Zusammensetzung nach einem der vorhergehenden Ansprüche, **dadurch gekennzeichnet, dass** sie außerdem einen oder mehrere Wirkstoffe, ausgewählt aus Weichmachern, Feuchthaltemitteln, Radikalfängern, entzündungshemmenden Mitteln, Vitaminen, Aufhellungsmitteln, Aknemitteln, anti-seborrhoischen Mitteln, Fungiziden, Keratolytika, Sonnenschutzmitteln, Verschlankungsmitteln, Hautfärbemitteln, enthält.

11. Medikament zur topischen Anwendung auf der Haut, umfassend eine Zusammensetzung nach einem der Ansprüche 1 bis 10.

12. Zusammensetzung nach einem der Ansprüche 1 bis 10 zur Verwendung bei der Behandlung von Psoriasis.

13. Kit oder einzelner Mehrkammerbehälter zur topischen Anwendung mit Spülen, der getrennt mindestens zwei Zwischenzusammensetzungen umfasst:
- eine Zwischenzusammensetzung A, umfassend mindestens ein Aktivierungsmittel für das Gaserzeugungsmittel, nach einem der Ansprüche 1 bis 10 und
- eine Zwischenzusammensetzung B, umfassend mindestens ein Gaserzeugungsmittel, nach einem der Ansprüche 1 bis 10;
- wobei Clobetasolpropionat in mindestens einer der Zwischenzusammensetzungen A und B enthalten ist.

14. Kit oder einzelner Mehrkammerbehälter nach Anspruch 13, **dadurch gekennzeichnet, dass** es bzw. er zum Mischen der Zwischenzusammensetzungen A und B in einem Gewichtsverhältnis A/B von 0,5 bis 2, bevorzugt von 0,5 bis 1,5, stärker bevorzugt von 0,9 bis 1,1 und noch stärker bevorzugt von 1 gestaltet ist.

15. Verfahren zur Herstellung einer Zusammensetzung in Schaumform zur topischen Anwendung ohne Spülen, enthaltend Clobetasolpropionat, wobei man eine Zwischenzusammensetzung A nach einem der Ansprüche 1 bis 10 mit einer Zwischenzusammensetzung B nach einem der Ansprüche 1 bis 10 in jeweiligen Gewichtsanteilen A/B von 0,5 bis 2, bevorzugt von 0,5 bis 1,5 und stärker bevorzugt von 1 mischt.

## Claims

1. A self-foaming composition containing clobetasol propionate, intended for rinse-off topical application, comprising:
- at least one intermediate composition B comprising a gas-generating agent and at least one gelling agent and/or suspending agent chosen from acrylic acid polymers, polysaccharides, the family of magnesium aluminum silicates, the family of modified starches and the family of carrageenans,
- at least one intermediate composition A comprising an agent for activating the gas-generating agent and at least one gelling agent and/or suspending agent chosen from polysaccharides, the family of magnesium aluminum silicates, the family of modified starches, the family of carrageenans and PVA, and
- clobetasol propionate contained in at least one of said intermediate compositions A and B.

2. The composition as claimed in the preceding claim, **characterized in that** clobetasol propionate is contained in the intermediate composition A.

3. The composition as claimed in either of the preceding claims, **characterized in that** it does not comprise any foaming surfactants, or that it contains such surfactants in a content of less than or equal to 2.5% by weight, preferably less than or equal to 1% by weight, relative to the weight of the total composition.

4. The composition as claimed in any one of the preceding claims, **characterized in that** the gas-generating agent is chosen from sodium bicarbonate, potassium bicarbonate, sodium carbonate and potassium carbonate, and mixtures thereof, and preferably the gas-generating agent is sodium bicarbonate.

5. The composition as claimed in any one of the preceding claims, **characterized in that** the gas-generating agent is present in the intermediate composition B in an amount ranging from 1% to 10% by weight and preferentially from 2% to 8% by weight, relative to the weight of the intermediate composition B.

6. The composition as claimed in any one of the preceding claims, **characterized in that** the agent for activating the gas-generating agent is chosen from an acid, a partially salified polyacid salt, a buffer solution of a weak acid and of its conjugate base, and mixtures of these compounds.

7. The composition as claimed in the preceding claim, **characterized in that** the agent for activating the gas-generating agent is chosen from citric acid, tartaric acid, malic acid, lactic acid, phosphoric acid and pyrophosphoric acid, and the salts of these acids, and more preferentially said activating agent is chosen from:
- a citric acid/sodium citrate buffer alone;
- phosphoric acid, sodium phosphate, disodium pyrophosphate, which are alone or as a mixture with a citric acid/sodium citrate buffer.

8. The composition as claimed in the preceding claim, **characterized in that** the agent for activating the gas-generating agent is a citric acid/sodium citrate buffer, alone or as a mixture with sodium phosphate and/or disodium pyrophosphate.

9. A composition in foam form, **characterized in that** it results from the mixing of said intermediate compositions A and B as claimed in any one of the preceding claims.

10. The composition as claimed in any one of the preceding claims, **characterized in that** it also contains one or more active agents chosen from emollients, humectants, free-radical scavengers, anti-inflammatory agents, vitamins, depigmenting agents, antiacne agents, antiseborrheic agents, antifungal agents, keratolytic agents, sunscreens, slimming agents and skin-coloring agents.

11. A medicament intended for topical application to the skin, comprising a composition as defined in any one of claims 1 to 10.

12. The composition as claimed in any one of claims 1 to 10, for its use in the treatment of psoriasis.

13. A kit or single multi-compartment container intended for rinse-off topical application separately comprising at least two intermediate compositions:
- an intermediate composition A comprising at least one agent for activating the gas-generating agent, as defined in any one of claims 1 to 10; and
- an intermediate composition B comprising at least one gas-generating agent as defined in any one of claims 1 to 10;
- clobetasol propionate being contained in at least one of said intermediate compositions A and B.

14. The kit or single multi-compartment container as claimed in claim 13, **characterized in that** it is designed for mixing the intermediate compositions A and B in an A/B weight ratio ranging from 0.5 to 2, preferentially from 0.5 to 1.5, more preferentially from 0.9 to 1.1 and even more preferentially 1.

15. A process for preparing a composition in foam form intended for leave-on topical application, containing clobetasol propionate, in which an intermediate composition A as defined in any one of claims 1 to 10 is mixed with an intermediate composition B as defined in any one of claims 1 to 10, in relative weight proportions A/B ranging from 0.5 to 2, preferentially from 0.5 to 1.5 and more preferentially 1.
